(19) Europäisches Patentamt
European Patent Office
Office européen des brevets

(11) **EP 1 462 775 B1**

(12) **EUROPEAN PATENT SPECIFICATION**

(45) Date of publication and mention
of the grant of the patent:
**05.12.2007 Bulletin 2007/49**

(51) Int Cl.:
*G01F 23/26* (2006.01)          *G01N 33/28* (2006.01)
*G01N 11/16* (2006.01)

(21) Application number: **04006925.4**

(22) Date of filing: **23.03.2004**

(54) **Device for detecting physical variables of lubricating oil of an internal combustion engine**

Vorrichtung zur Detektion physikalischer Variablen von Schmieröl eines Verbrennungsmotors

Dispositif pour détecter des variables physiques de l'huile lubrifiante d'un moteur à explosion

(84) Designated Contracting States:
**AT BE BG CH CY CZ DE DK EE ES FI FR GB GR HU IE IT LI LU MC NL PL PT RO SE SI SK TR**

(30) Priority: **25.03.2003 IT TO20030219**

(43) Date of publication of application:
**29.09.2004 Bulletin 2004/40**

(73) Proprietor: **ELTEK S.p.A.**
**I-15033 Casale Monferrato (Alessandria) (IT)**

(72) Inventors:
• **Gadini, Costanzo**
  **15040 Frassineto Po (Alessandria) (IT)**

• **Zorzetto, Mauro**
  **15033 Casale Monferrato (Alessandria) (IT)**
• **Boverio, Alessandro**
  **15033 Casale Monferrato (Alessandria) (IT)**

(74) Representative: **Gallarotti, Franco**
**Buzzi, Notaro & Antonielli d'Oulx Srl,**
**Via Maria Vittoria, 18**
**10123 Torino (IT)**

(56) References cited:
**WO-A-02/084228          DE-A- 10 161 918**
**DE-A- 10 202 030          DE-A- 19 706 486**
**US-A- 5 051 921          US-B1- 6 433 560**

## Description

The present invention relates to a device for detecting physical variables of a fluid, comprising capacitive type sensor means.

Devices for detecting with capacitive type sensor means are for example used to detect certain quantities relative to the lubricant oil of an internal combustion engine, such for example amount, degree of deterioration, etcetera.

More in particular, the level capacitive sensors comprise two electrodes or plates (armatures), which form a capacitor, between which the fluid to be controlled and monitored flows; the functioning of such sensors is based on the principle that the electric capacity between two capacitor's plates is described by the formula

$$C = \varepsilon * S * (N-1)/d$$

where "S" is the area of the opposite surfaces of the plates, "N" is the number of the plates, "d" is the distance between the plates and "$\varepsilon$" is the dielectric constant of the fluid.

A fluid which level increases between the two plates determines an increase of the measured capacity. However, a possible variation of the dielectric constant of the fluid which separates the opposite surfaces of the plates determines a change of the measured capacity; such a change of capacity due to a variation of the dielectric constant of the fluid could therefore be by mistake attributed to a change of level. For this reason, in the devices of the type indicated it is common practice to foresee, in addition to the capacitive level sensor, also a capacitive reference sensor having the function to detect the dielectric constant of the fluid; in such a way the level reading can be suitably corrected, in the case of variations of the dielectric constant of the fluid. Such capacitive reference sensor results in being constantly immersed in the fluid; for this reason it is usually placed in the lower part of the fluid container.

A device of the type previously mentioned is known by document WO02084228 upon which the preamble of claim 1 is based. The device here described foresees a detecting probe comprising of a first and second electrode obtained from a metallic strap, that is to say from a relatively thin metal sheet, and therefore of a flat form; the plate surface of the capacitor is defined in the thickness of the metallic strap and is substantially "comb" like, that is to say having a complex profile apt to increase the extent of such sur-face. A plastic frame keeps the two electrodes in relative fixed positions, in a co-planar way and with the plate surfaces facing each other, with the teeth, i.e., the protruding profile, of an electrode situated in the spaces, i.e., in the indented profile, which separate two successive teeth of the other electrode, and vice versa, without ever getting in contact. The first and second mentioned electrodes are part of a capacitive sensor provided to monitor the level of the engine oil.

The device further includes a second capacitive sensor, provided to detect the dielectric constant of the fluid; the second sensor includes a third comb-shape electrode, that is to say analogous to that described previously, also obtained from a metallic strap, having a reduced height with respect to the other two, placed below the first electrode and kept in fixed position with respect to the second electrode through the said plastic frame. The third electrode is co-planar with respect to the first and the second electrode and is placed in such a way that its teeth are interposed, without contact, with a set of teeth of the second electrode. According to what has been said, the second electrode is electrically connected in common to the first and third electrode, yet realizing two separate capacitor's plates, of equal structure or form between them, even if with different dimensions.

US-A-5 051 921 discloses a liquid level and composition sensor which includes a first interdigitated capacitor mounted substantially vertically in a tank, and a second interdigitated capacitor mounted substantially horizontally in and near the bottom of the tank. An electronic processor is responsive to the value of capacitance of the first capacitor for producing a first voltage signal proportional to the level of the liquid in the tank and is responsive to the value of capacitance of the second capacitor for producing a second voltage signal having a voltage level corresponding to the composition or dielectric constant of the liquid The processor also multiplies the first and second voltage signals together to produce a liquid level voltage output signal having a constant slope for voltage amplitude versus liquid level, regardless of the composition of the liquid in the tank.

Practical tests carried out by the applicant have allowed to establish that, in the detecting devices of known type, the production's tolerances, the ageing and the changes of the electric characteristics of the plastic materials used to realize the above said frame, in the range of temperatures of use cause variations in the dielectric constant and/or in the capacity, introducing those which are also defined as parasitic capacities of the capacitor, with consequent measuring errors.

In view of that above, the present invention intends to realize a detecting device of the type previously indicated having a new and advantageous structure, being of simple realization and economically convenient, and also of functioning more precise and reliable in the course of time.

These and other aims also are achieved, according to the present invention, by a detecting device having the characteristics of claim 1.

Further aims, characteristics and advantages of the present invention will result clear from the detailed description which follows and from the enclosed drawings, supplied purely as an explanatory but not limitative example, in which:

- figures 1, 2, 3 and 4 are respectively a lateral view, a frontal view, a perspective view and a plan view of a device according to the invention;
- figures 5, 6, 7 and 8 are respectively a lateral view, a frontal view, a perspective view and a plan view of a detecting probe being part of the device of figure 1;
- figures 9, 10, 11 and 12 are respectively a perspective view, a lateral view, a frontal view and a plan view in a bigger scale of an electrically conductive part of the probe to which figures 5-8 refer;
- figures 13, 14 and 15 are frontal views of components of the conductive electrical part to which figures 9-12 refer;
- figure 16 is a frontal view of some of the components to which figures 9-12 refer as obtained from a same metallic strap;
- figures 17, 18 and 19 are respectively a perspective view and two enlarged details of a mould utilized with the aim to realize the probe to which figures 5-8 refer;
- figures 20 and 21 are respectively a perspective view and an enlarged detail of the mould to which figures 17-19 refer, with the components inserted to which figures 13-15 refer;
- figures 22 and 23 are respectively a perspective view and an enlarged detail of the mould to which figures 17-19 refer, following the realization of the probe to which figures 5-8 refer;
- figures 24, 25, 26 and 27 are frontal views of components of the electrically conductive part of a probe being part of a detecting device according to a further possible variation of the invention;
- figures 28 and 29 are respectively a perspective view and an enlarged detail of a probe which includes components to which figures 24-27 refer, during its assembly;
- figures 30 and 31 are respectively a perspective view and an enlarged detail of the probe to which figures 28, 29 refer, during a successive phase of its assembly;

In figures from 1 to 4, 1 indicates as a whole a device for detecting physical variables of a fluid, according to the invention. In the example, the device 1 is provided for detecting variables relating to an oil used in an internal combustion engine, such variables being in particular represented at least by the oil level, its temperature, its density and its degree of deterioration.

The device 1 includes a supporting body 2 in electrically insulated material, preferably molded in thermoplastic material, in which peripheral seats are defined, destined to lodge respective metallic bushings 4; body 2 has a lateral tubular portion 5 which, jointly with respective internal terminals for connection, some of which indicated with TC, form a connector to connect or to interface the device 1 to a control system, for example of a vehicle, not shown.

Body 2 includes an upper part 11A, being substantially cylindrical or conical frustum shaped, from the top of which a prismatic portion 11B rises in an orthogonal way. On body 2, or to the top of part 11A, a probe 20 is anchored, to detect the level of the oil and its degree of deterioration, as will be described later.

As visible in figures from 5 to 8, probe 20 has a supporting frame in electrically insulated material, preferably a thermoplastic material, indicated as a whole with 21, defining a base 21A, two parallel longitudinal uprights 21B, 21C, an intermediate cross piece 21D and a top cross piece 21E. Frame 21 is provided to support and to keep in respective fixed positions three electrodes of metallic material obtained from a metal strap, indicated as a whole with 22,23 and 24, later on described in detail, each one having a respective connection end shaped as a flat terminal 22A, 23A and 24A. From base 21A of frame 21 a first coupling appendix 25 departs in an orthogonal way downwards, from which the terminal 22A extends, and a second coupling appendix 26, from which the terminals 23A and 24A extend; the external part of the appendices 25 and 26 is shaped to define a series of teeth or knurlings, suitable to improve the coupling and/or the sealing with the base 2. From each of the smaller sides of base 21A of frame 21 a respective lateral hooking tooth 27 departs in an orthogonal way upwards, the teeth 27 being provided to lock in position a cover of the device, or a part of the body provided with suitable ducts for the entry and exit of the fluid. From each one of the smaller sides of base 21A of frame 21 two rest extensions 28 depart in an orthogonal way towards the outside.

In figures from 9 to 12 only the mechanical part of the probe 20 is represented, made up of the said electrodes 22, 23 and 24; in figures from 13 to 15 such electrodes 22, 23 and 24 are singularly visible, that is to say as obtained starting from at least a metallic strap of reduced thickness, suitably cut and shaped.

Electrode 22 has an extended longitudinal portion 22B, to the base of which the respective terminal 22A is defined; from portion 22B, a series of first teeth or projections 22C departs laterally, shaped substantially in a triangular or pointed way, that is to say having a shape that narrows towards the external end, and a small plaque portion 22D, of substantially rectangular shape, which is located in the lower part of electrode 22, occupying a reduced part of the longitudinal development of the same electrode; teeth 22C are formed instead in the remaining upper area of electrode 22, occupying the prevailing part of its longitudinal development. As can be seen, in the ambit of the area of electrode 22 occupied by the small plaque portion 22D a through slot 22F is defined, which extends longitudinally in the direction of the height of electrode 22.

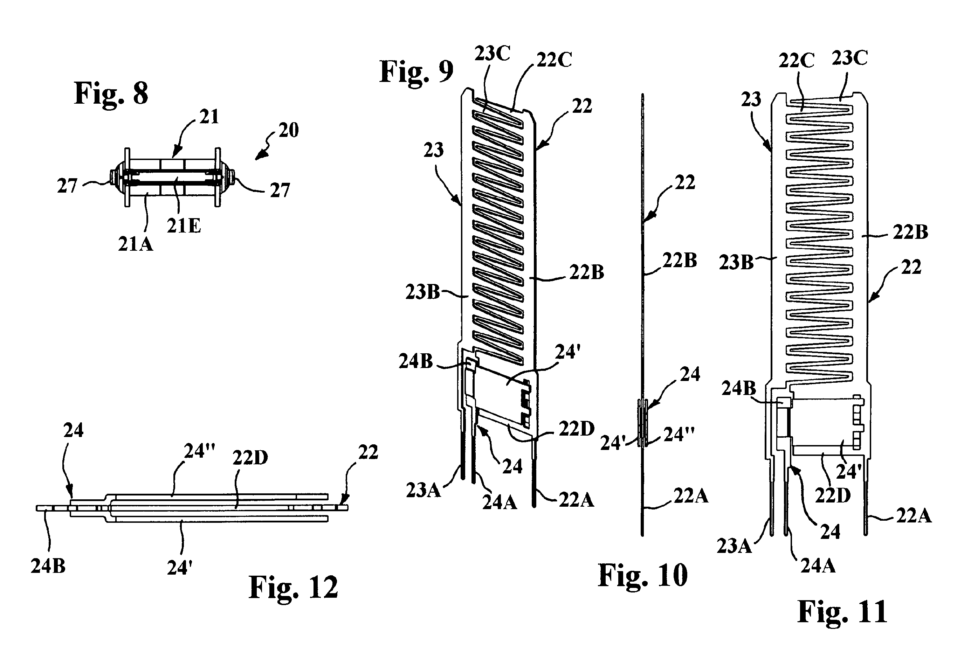

Electrode 23 has a total length substantially equal to that of electrode 22 and presents a longitudinally extended portion 23B from which a single series of teeth or projections 23C departs laterally of analogous form to teeth 22C of electrode 22. Teeth 23C are formed in the upper part of electrode 23, in an area substantially homologous to the one in which teeth 22C are defined in electrode 22, but in a slightly staggered position in regard to these last ones. In such a way, when the two electrodes 22, 23 are coplanar, in the operative position within the frame 21, teeth 22C are interdigitated with teeth 23C, without ever getting in contact, as clearly visible, for example, in figures 9 and 11. Teeth 22C, 23C, or better the respective peripheral surface obtained in the thickness of the metallic strap, realize the plate surfaces of the capacity sensor aimed to detect the oil level.

As already seen, electrodes 22 and 23 are each one realized in a single piece of metallic strap, and therefore in the form of a lamina having two main faces, or faces of greater surface, being parallel to each other, between which the thickness of the lamina or of the metallic strap itself is defined; the surface corresponding to said thickness forms the plate of the capacitor or detecting probe. In the exemplified embodiment, electrode 24 is instead made of assembled parts, and these are also in the form of a lamina obtained from a metallic strap, with two main parallel faces defining the thickness of the lamina itself. Electrode 24 is of a length shorter than electrodes 22 and 23 and presents a longitudinally extended portion 24B having a development substantially equal to that of the part of the portion 22B of electrode 22 in which the small plaque portion 22D is defined.

Electrode 24 further includes two metallic small plaques 24' and 24"; each plaque 24', 24" has a main portion 24G, substantially rectangular, from the outer edge of which two positioning extensions 24H depart; from the opposite edge a fixing extension 24I departs; as can be noted in figure 12, the extension 24I of each plaque 24', 24" lies on a different level, substantially parallel compared with that on which the respective principal portion 24G lies.

For the manufacturing of electrode 24, extensions 24I of the two plaques 24', 24" are welded, each one on a respective face of portion 24B; the welding could take place through a classic operation of spot welding, in such a way that the two plaques 24' and 24" become mechanically and electrically connected to portion 24B. In this way, electrode 24 becomes substantially formed by portion 24B, defining in the lower part the terminal 24A, from which depart laterally, in a substantially orthogonal way, the two plaques 24', 24", substantially parallel between them and electrically connected, to form a U section.

When electrodes 22, 23 and 24 are kept in the respective operative portions through frame 21, in the U shape space defined between the portions 24G of the two plaques 24', 24", the plaque portion 22D being part of electrode 22 extends, without the second getting in contact with the first ones, as visible for example in figure 12. As previously mentioned, frame 21 is provided for such purpose to assure a firm relative position between the electrodes 22, 23, 24, without these ever getting in direct contact between them.

The plaque portion 22D of electrode 22 and electrode 24 form a capacitive sensor to measure the dielectric constant of the oil; such capacitive sensor includes in this case four plates surfaces, faced between them in pairs, formed in particular by the two faces of portion 22D and by the inside faces of plaques 24', 24". As can be noted, on the said capacitive sensor of reference the plates surfaces are each one defined by a main face, or a face having a bigger area, of the lamina which forms electrode 22 and of the lamina which form the plaque 24', 24" of electrode 24; vice versa, in the case of the measuring capacitive sensor formed by electrodes 22, 23 the "toothed" plate surfaces are formed in the thickness of the respective lamina.

Going back to figures from 1 to 4, probe 20 is anchored to body 2, at the top of part 11A, extending in a direction substantially coinciding with that of prismatic portion 11B. To such aim, probe 20 can be fitted with interference in part 11A and there locked in a known manner; this last operation can be substantially accomplished by inserting the appendices 25 and 26 into respective passages of part 11A of body 2, from the side where the prismatic portion 11B elevates. The interference between the indentation or knurling present on the external surface of the appendices 25, 26 with said passages permits to obtain a mechanical coupling between the two components 20 and 10; such coupling could eventually be completed in a known way, for example with glue, sealers, re-melting of material, etc. The presence of the mentioned indentation has also the function to obtain a sort of labyrinth, suitable to prevent eventual infiltration of oil through said passages.

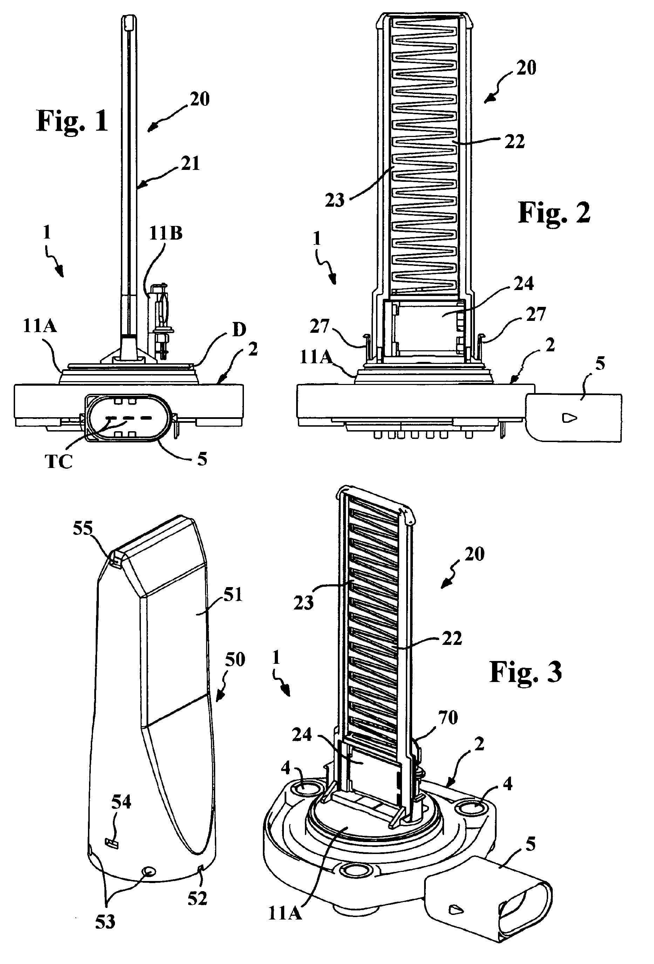

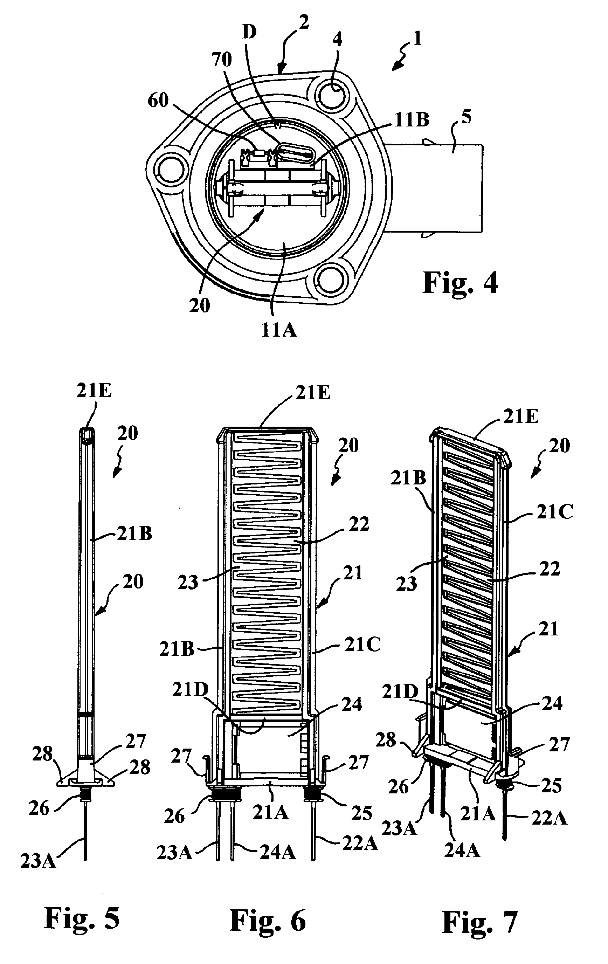

On probe 20 a cover is meant to be mounted, indicated with 50 in figure 3; cover 50 has a body 51, for example in molded thermoplastic material with a part of the base being tapered, an intermediate part of substantially constant section and a head part being pointed. In the base part of body 51, in correspondence with its lower edge, at least a seat or groove 52 is defined, which is meant to cooperate with a respective relief D of part 11A of body 2 (see figure 1 and 4); in proximity of said lower edge holes 53 are also defined for the passage of the oil, and seats or slits 54, each one meant to cooperate with a respective tooth 27 of probe 20. In the head portion of body 51 further passages 55 are also defined for the fluid and/or as a vent.

Also in figure 4, with 60 a temperature sensor is indicated, constituted in the specific case by a negative temperature coefficient resistor, or NTC. With 70 an electronic generator of oscillations is indicated, constituted in the specific case by a quartz.

Sensor 60 and quartz 70 are electrically connected to terminals embedded inside body 2, having first ends projecting from the prismatic portion 11B, and second ends electrically connected to terminals TC of connector 5; analogous considerations are valid for terminals 22A, 23A and 24A of electrodes 22, 23 and 24. The methods of electrical connection of components 22-24, 60, 70 to terminals TC and for interfacing to the respective control circuit are not part of the present invention and, as these can be of any known type, they will not be illustrated and described here.

To the lower part of body 2 a shell or lower closing body is meant to be fixed, not represented here, having a configuration substantially concave and realized in molded thermoplastic material.

The detecting device 1 obtained as previously described is meant to be fixed, for example by screws passing through the bushings 4 of body 2, to the lower part of a container of the oil to be monitored (for example an oil sump), in correspondence to a respective opening, in such a way that the entire cover 50 fits inside such container. The fixing method of the device 1 and the means to secure the hermetic seal between the body of the same and the oil container are not described here, because these could be of a known type. The oil held in the container equipped with the device 1 can penetrate and/or exit through holes 53 and/or 55 inside the cavity of the cover, to get in contact with the probe 20, the sensor 60 and the quartz 70.

As previously mentioned, electrodes 22 and 23 are part of a first sensor, in particular a capacitive sensor and/or of electrical conductivity, operating according to a known technique for detecting the oil level. In brief, electrodes 22, 23 de facto make up the plates of a capacitor, between the opposite surfaces of which (that is to say the surfaces made by teeth 22C, 23C) is established a certain electrical capacity, when the same are immersed in the oil and between them a current is applied; following the eventual reduction of the oil quantity inside the respective container, the part of the electrodes 22-23 immersed in the oil is reduced, this determining a different electrical capacity between the same electrodes. Such variations of capacity can be evaluated electronically in a known way, in order to determine the oil level. Substantially on the base of the same principle the arrangement of which electrodes 22, 23 are part, allows also to have information concerning the degree of degradation of the oil (to such regard it should be considered that the conductivity of the oil increases with the respective ageing).

As illustrated, in the preferred embodiment of the invention teeth 22C, 23C of electrodes 22, 23 are substantially of a triangular or pointed shape, or with a form defined by two sloped planes that meet each other; this with the aim to ease the slipping or dripping of the oil from the same teeth, so avoiding false readings.

The capacitor of which electrode 24 and the lamina 22D of electrode 22 are part has mainly the function to detect the dielectric constant of the oil, as a reference for measuring the level; in other words, the above mentioned capacity of reference supplies, every time a detection is carried out by the device 1 during its functioning in the car, the starting point of the recognition line relating to the capacitive sensor of which electrodes 22, 23 form part (capacity in function of the level) according to the characteristics of the oil and of the temperature, detected through sensor 70.

As mentioned, at the base of the present invention there is the acknowledgement of the fact that the variation of the parasitic capacities of the capacitors, due for example to production tolerances, ageing and the change with time of the electric characteristics of the plastic materials used to keep the electrodes in position, determines mistakes of readings.

Such a problem is solved, according to the invention, making sure that the capacity of the reference capacitor be much bigger than that of the parasitic capacities. In such a way the error due to the variation of the parasitic capacities, for the causes above specified, is reduced. The performance of the reference capacitor comprising electrode 24 and part 22D of electrode 22 are improved in that sense through a geometry characterized by the presence of opposing capacitor's plates well extended in regard to the previously cited prior art.

To such regard it has to be remembered how, in the detecting devices with coplanar flat armatures (see cited WO02084228) the opposed surfaces of the plates of the reference capacitor are of reduced size, since said size is limited by the face defining the thickness of the metallic strap from which each plate is obtained. On the contrary, in the case of the present invention, and as it can be deduced from example from figure 12, the reference capacitor includes electrodes with opposed plate surfaces much larger with respect to the prior art; this because such plate surfaces are realized by the faces of parts 24G, 22D being perpendicular with respect to the thickness of the metallic strap from which the same parts 24G, 22D are obtained.

The reference capacitor of device 1 according to the invention may therefore have opposite or parallel faces very extended, substantially with equal available space for its placing in regard to the known technique. Furthermore, in the case exemplified in the drawings, the opposite faces of the reference capacitor are even four, this constituting a possible further more accurate measurement.

Regarding sensor 60 and quartz 70, these are positioned in an area near body 2, similarly to electrode 24, and therefore near of the bottom of the oil container, in such a way to be able to operate also in condition of low level and/or to detect the characteristics of the fluid near the capacitive sensor.

As mentioned, sensor 60 is provided for detecting the temperature of the oil. The variable detected by sensor 60, besides having importance in absolute terms (that is to say in knowing the oil temperature) is also used with the aim to "compensate" the detections made through probe 20 and/or quartz 70. This in consideration of the fact that some physical characteristics of the oil could change at different temperatures of the fluid; for example, the viscosity value of the oil at a first temperature is different from that one detectable at a different working temperature of the same fluid.

In such light, therefore, in the case in which the values detected through probe 20 and/or quartz 70 must be compared with data or fixed reference tables, the control logic which supervises the functioning of device 1 will operate to compensate such detected values in relation to the oil temperature at the time of detecting the interested quantity, as measured by sensor 60. Alternatively, the control logic might foresee that determined detecting phases through the probe 20 and/or quartz 70 will take place when, the oil reaches a predetermined temperature, such attainment being detected by sensor 60.

The oscillation generator or quartz 70 is used with the aim to measure the oil viscosity, according to known methods, on the base of the fact that the quartz varies its oscillation frequency in relation to the oil viscosity. Since viscosity depends in a certain measure on the temperature of the fluid, in the preferred form of the invention temperature sensor 60 is positioned as near as possible to quartz 70, to have correct information concerning the viscosity and therefore the degradation of the oil.

It should be noted that the electronic components called generically "quartz" usually include a protective cover of a respective quartz crystal, connected to two electric contacts, that when the electric current passes through it oscillates at a certain frequency. In the case of the present invention, component 70 is preferably without said cover, and therefore formed solely by the quartz crystal, with the respective protruding terminals. That allow to further increase the precision of measurement effected through quartz 70. Nothing obviously impedes to foresee a cover with holes for the quartz, for example with a form similar to a net.

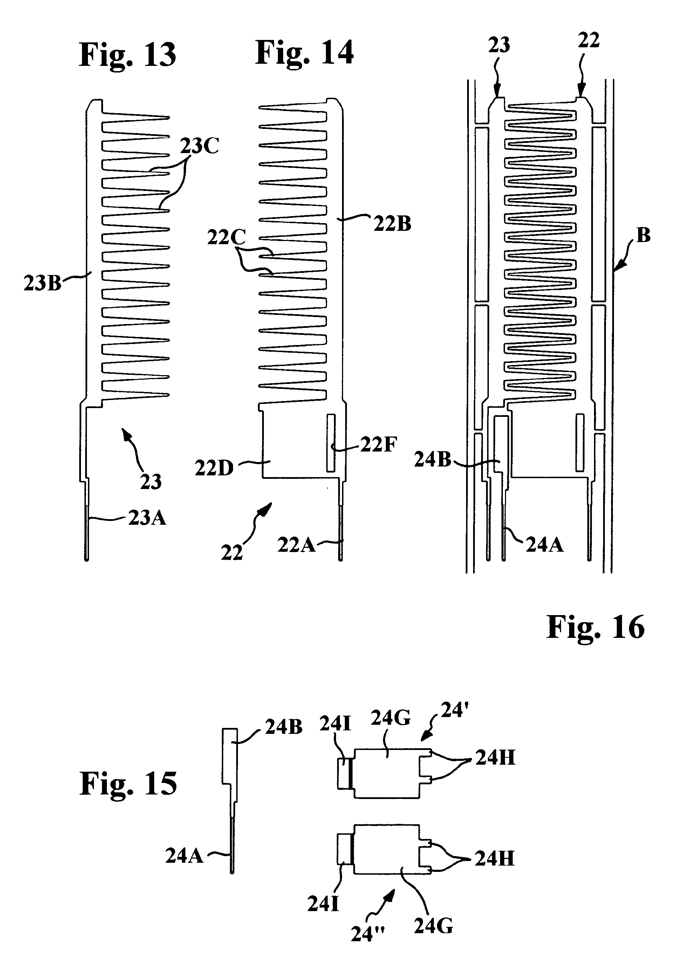

Probe 20 is made by over-molding plastic material on the elements of electrically conductive material shown in figure 13-15, cut from a metallic strap. To such aim a stainless steel strap is sheared to obtain electrodes 22, 23 and 24, and also the small plaques 24' and 24". As visible in figure 16, at the end of the said shearing operation, from the original metallic strap B the electrodes 22, 23 and the portions 24A-24B of electrode 24 are formed. After a first shearing, components 22, 23 are still connected, through small bridges or elements of connection, to residual parts of the original metallic strap B; once sheared and/or eliminated the said residual parts of the metallic strap and the respective small bridges, the separated components 22, 23 and 24A - 24B are available. During said phases of production, to component 24A - 24B the two small plaques 24', 24", previously obtained with known methods (for example also these through shearing of a metallic strap) are also welded. Obviously the sequence of manufacturing could be different, that is to say with the various parts firstly welded and then sheared, through an automatic system which welds, shears and positions afterwards the made up component into a mold.

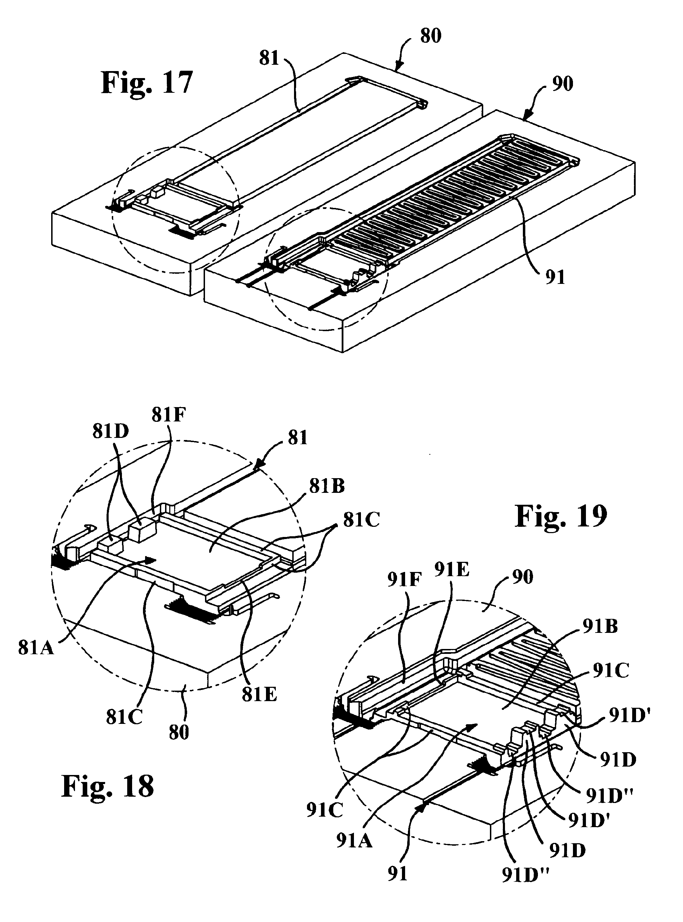

In figure 17, with 80 and 90 two half molds are indicated, in each one a respective impression 81, 91 being defined; the two half molds are used to mold over the said metallic components 22-24 the thermoplastic material destined to realize the frame 21 of probe 20.

In the ambit of impressions 81 and 91 proper hollow zones are defined, for positioning the metallic parts destined to remain directly exposed to the oil, and in particular teeth 22C, 23C, small plaque portion 22D of electrode 22 and the main portions 24G of the small plaques 24', 24" of electrode 24.

In particular, as it is noted in the detailed view of figure 18, in the impression 81 a zone 81A is formed, delimited by a bottom surface 81B and lateral walls 81C; in the zone 81A two parallel projections 81D rises from the surface 81B; in the wall 81C which is situated on the opposite side to said projections an undercut 81E is defined. In the impression 81 a positioning zone 81F is also foreseen for portion 24B of terminal 24 and for extension 24I of the associated small plaque 24'. In the same way, in the impression 91 a zone 91A is defined, delimited by a bottom surface 91B and lateral walls 91C; in the zone 91A two parallel projections 91D rise from surface 91B, to the top of which positioning seats 91D' are formed; between the two projections 91D seats 91D" are formed of analogous shape to those of seats 91D'; also in this case, in wall 91C on the opposite side to the extensions 91D an undercut 91E is defined and in the impression 91 a positioning zone 91F is foreseen for a respective part of portion 24B of terminal 24 and of the extension 24I of the associated small plaque 24".

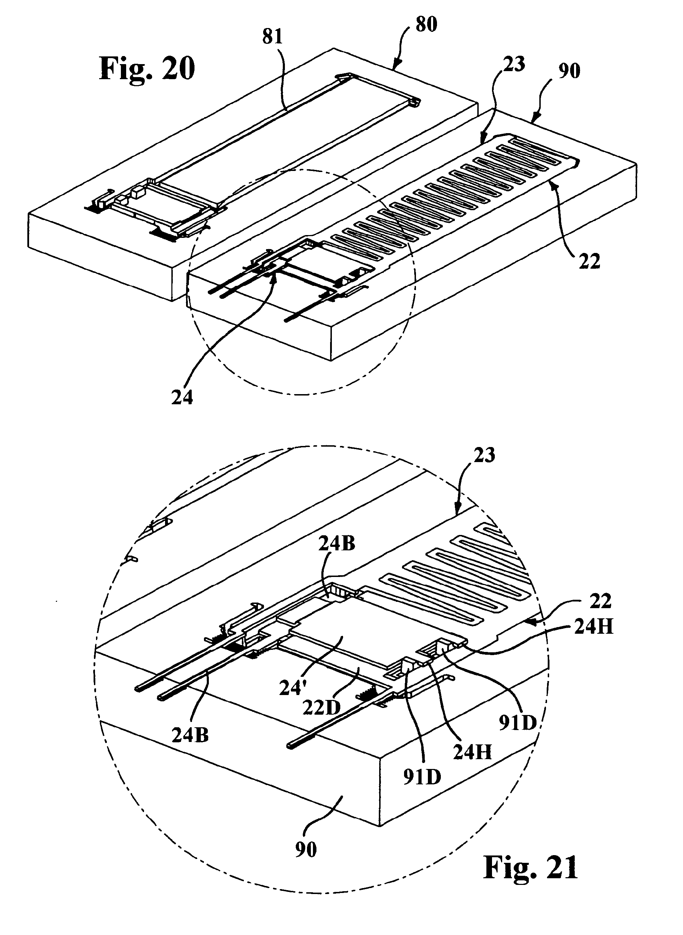

For the manufacturing of frame 21, electrodes 22, 23 and 24 are placed, with the configuration illustrated in drawings 9 - 12, in the half-mould 91; such operative phase is visible in figures 20 and 21. A part of portion 24B of terminal 24, with the extension 24I of the respective small plaque 24" is thus positioned in the ambit of area 91F (figure 19), in such way that the external surface of portion 24G of the small plaque 24" is in contact with the bottom surface 91B of zone 91A, and with the extension 24I in correspondence of the undercut 91E; extensions 24H of the small plaques 24', 24" are positioned respectively in the seats 91D' and 91D" (see also figure 19); projections 91D, on which the seats 91D' are defined, are passing through slot 22F of the small plaque portion 22D of electrode 22, as visible in figure 21. When the half-mould 80 is then closed on the half-mould 90

- surface 81B of impression 81 is in contact with the external surface of portion 24G of small plaque 24', with extension 24I of the same small plaque in correspondence to the undercut 81E;
- a part of portion 24B of electrode 24, with the respective extension 24I of the small plaque 24' results in the positioning zone 81F,
- projections 81D are pressed on extensions 24H of small plaque 24", between projections 91D.

Through such disposition, therefore the correct relative positioning is assured between the small plaques 24', 24" and the small plaque portion 22D the electrode 22; the free space between the two small plaques 24', 24" and a respective face of the lamina portion 22D becomes closed on every side, by means of walls 81C, 91C, so to avoid its filling up with the molding material.

In the closed mould 80 - 90 the thermoplastic material is injected, with known methods, which fills the impressions 81 - 91 facing each other, having the metallic components 22, 23, 24 inside.

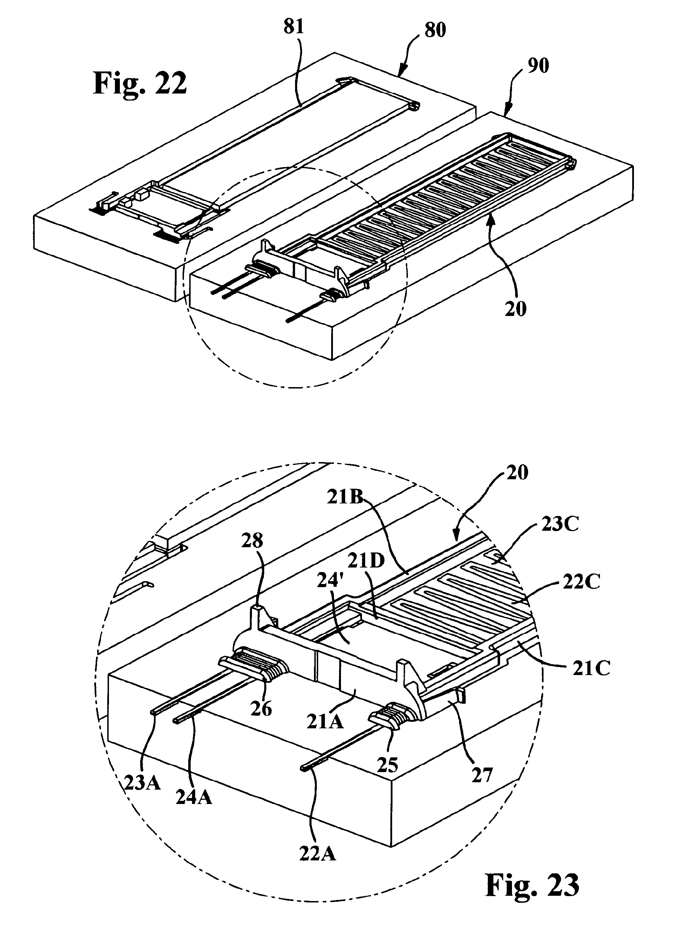

At the end of the injection molding operation, the half moulds 80, 90 are separated, for the extraction of probe 20 by this time formed, as visible in the figures 22 and 23.

The preferred thermoplastic material for the realization of body 2 of the device 1 and of frame 21 is the poly-phenylene sulphide or PPS. Such material, internally reinforced and lubricated, presents an excellent combination of properties, as a load-carrying capacity, wear resistance and dimensional stability also in the case of exposure in an ambient characterized by chemical agents and high temperatures. PPS, besides, compared with a nylon, has the characteristic of not being "porous" and/or not to "absorb" liquid; with the consequence that also the dielectric characteristics are improved.

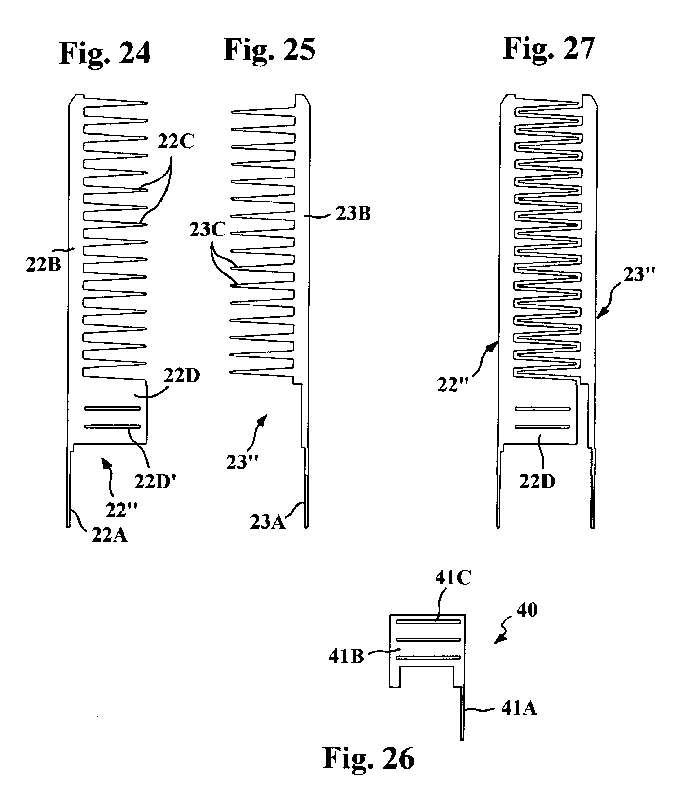

In figures 24-31 there are visible the metallic parts of a capacitive sensor probe of a detecting device realized according to a further embodiment of the invention.

As it is evident in figures 24 and 25, for that scope two electrodes 22", 23" are provided, having a shape substantially similar to that one of electrodes 22, 23 of figures 13, 14, obtainable through a metallic strap; in figures 38, 39 the same numerals of figures 13, 14 are used, to indicate elements technically equivalent.

In this case, the small plaque portion 22D of electrode 22" is provided with transversal slots 22D'. The probe further includes a third electrode, indicated as a whole with 40 in figure 26, itself also obtained starting from a metallic strap and therefore of flat configuration; terminal 40 includes a part shaped as a terminal 41A and a part shaped as a small plaque 41B, provided with through slots 41C. As for the case of probe 30 previously illustrated, the number of slots 22D' of electrode 22" differs by the number of slots 41C of electrode 40.

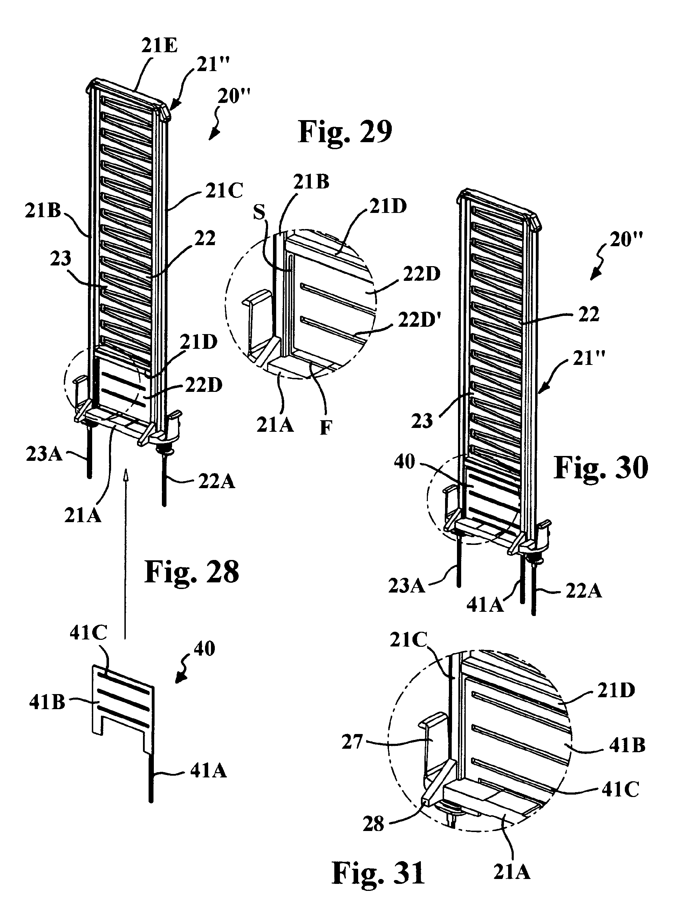

In figure 27 there is visible the relative fixed position that the two electrodes 22" and 23" must have within the respective thermoplastic frame; such frame, integrating the respective terminals 22", 23" is visible in figure 28, where is indicated by 21".

Frame 21" is of a realization substantially similar to frame 21 of probe 20 previously described. In this case, as it may be noted on the enlarged detail of figure 29, frame 21" is molded in such a way to present at least a seat or a through slot F, in its base or bottom cross piece 21A, from which respective seats or lateral guides also depart on the uprights 21B, 21C, one of such guides being visible in S; such guides S are extending on the uprights of frame 21" substantially between the lower cross piece 21A and the intermediate cross piece 2 1 D of frame 21".

As it can be perceived in figure 28, electrode 40 is driven from the bottom in the frame 21", that is to say put through with slight pressure inside slot F, and made to slide inside guides S until it reaches the position visible in figures 30, 31; in such position, the small plaque part 41B of electrode 40 is substantially parallel and spaced apart in respect of the small plaque portion 22D of electrode 22". Eventually the third electrode 40 could be kept in position, instead of by pressure, with any other equivalent solution, such as, for example, thermo-fusion of material, vibration welding, mechanical lock, resin finish, etc.

From the description made, and also by the enclosed claims which form an integral part of it, the characteristics of the present invention become clear, as its advantages also become clear.

Trial tests carried out have ascertained that the detecting devices realized according to the invention, having reference capacity sensors of large opposite surfaces, solves the drawbacks declared in the introductory part of the present description, and still safeguarding the strength and compactness of the device.

The reference sensor of the device according to the invention has also shown to be useful to zero, in the phase of initial calibration on the assembly line, the offset of each sensor of the device determined by the parasitic capacities. It should be noted that such parasitic capacities are not constant at the variation of the pieces, but vary in function of the distance of the metallic straps, of the thickness of the plastic between the various terminals, etcetera; the said zero setting is preferably actuated in the production phase using an oil sample.

It is clear that numerous variations are possible, for the man skilled in the art, to the device described as an example, without leaving from the inventive idea, as defined in the appended claims.

The device 1 according to the invention could be advantageously used in fields different from the one mentioned simply as a non limitative example. In other possible variations of embodiments and/or of use of the device according to the invention, the surfaces of the capacitor's plate formed in the thickness of a respective lamina could be those of a reference sensor, and the surfaces of the capacitor's plate defined in a main face of a respective lamina could be those of the measuring sensor.

## Claims

1. Device for detecting physical variables of a fluid having a dielectric constant, the device (1) including a body (2) associated with at least

   - a first sensor element, of the type comprising a capacitor, to detect at least the level of the fluid inside a respective container, and
   - a second sensor element, of the type comprising a capacitor, to detect at least one parameter of reference of the fluid, such as its dielectric constant,

wherein

- each sensor element has at least two electrodes (22, 23, 24; 22", 23", 40) obtained starting from at least one metallic lamina having two main faces and a peripheral surface which is defined by the thickness of the lamina, each electrode (22, 23, 24; 22", 23", 40) defining at least a respective capacitor's plate surface facing an homologous capacitor's plate surface of the other electrode (22, 23, 24; 22", 23", 40),
- the electrodes (22, 23;22", 23") of one of said first and second sensor element have at least a respective capacitor's plate surface which is formed in the peripheral surface of a respective lamina, the electrodes of said one sensor element comprising a first and a second electrode (22, 23; 22", 23") of the device (1), the capacitor's plate surface of each of said first and second electrode (22, 23; 22", 23") having a plurality of projections (22C, 23C) sequentially arranged along a longitudinal extension of the electrode, said first and second electrode (22, 23; 22", 23") being arranged such that the projections (22C) of the first electrode (22; 22") are co-planar to, and interdigitated with, the projections (23C) of the second electrode (23; 23"),
- the first and second sensor element have at least in common said first electrode (22; 22"),

**characterized in that**

- the electrodes (22, 24; 22", 40) of the other of said first and second sensor element have at least a respective capacitor's plate surface defined in a main face of a respective lamina, the electrodes (22, 24; 22", 40) of said other sensor element comprising a third electrode (24; 40) of the device (1), the third electrode (24; 40) comprising at least one plaque portion (24', 24"; 41B) with a main face thereof which realizes a respective capacitor's plate surface of said other sensor element;
- said first electrode (22; 22") also comprises a plaque portion (22D) having at least a main face which realizes a respective capacitor's plate surface of said other sensor element, and
- the first and the third electrodes (22, 24; 22", 40) are arranged such that the respective plaque portions (22D, 24', 24"; 22D, 41B) are substantially parallel or uniformly spaced apart to each other.

2. Device according to claim 1, **characterized by** comprising a supporting structure made of an electrically insulated material (21, 21 "), designed

- to keep substantially on a same lying plane the first and the second electrodes (22, 23; 22", 23"), and
- to keep said at least one plaque portion (24', 24"; 41B) of the third electrode (24; 40) substantially parallel or uniformly spaced apart to the plaque portion (22D) of the first electrode (22; 22").

3. Device according to claims 2, **characterized in that** the supporting structure is made of a thermoplastic material molded over the first, the second and the thirds electrode (22, 23, 24).

4. Device according to claim 1, **characterized in that** the plaque portion (22D) and the plurality of projections (22C) of the first electrode (22; 22") lie on a same plane.

5. Device according to claim 1, **characterized in that** the third electrode (24) comprises two plaque portions (24', 24"), between which the plaque portion (22D) of the first electrode (22; 22") is placed.

6. Device according to claim 5, **characterized in that** the third electrode (24) is formed by a number of separated parts (24A-24B, 24', 24"), connected electrically and mechanically to each other.

7. Device according to claim 6, **characterized in that** at least some of said separated parts (24A-24B, 24', 24") are reciprocally welded.

8. Device according to claim 1, **characterized in that** at least one of said plaque portions (22D, 24', 24"; 22D, 41B) has at least a respective through slot (22D'; 41C).

9. Device according to claim 1, **characterized in that** said projections (22C, 23C) are shaped like pointed teeth.

10. Device according to claim 1, **characterized in that** said one sensor element comprises a frame (21") for the first and second electrodes (22", 23") defining seats or guides (F, S) in which the third electrode (40) is at least partially inserted or fixed.

11. Device according to claim 1, **characterized in that** sensor means to detect the temperature (60) of the fluid are further associated to said body (2).

12. Device according to claim 1, **characterized in that** sensor means to detect the viscosity of the fluid are further associated to said body (2), comprising a generator of oscillation (70).

**13.** Device according to claim 2, **characterized in that** the supporting structure (21;) comprise at least two parallel longitudinal uprights (21 B, 21 C) and a cross piece (21A, 21D, 21E).

**14.** Device according to claim 13, **characterized in that** the supporting structure comprises a base cross piece (21A) from which at least two extensions for coupling (25, 26) to said body (2) depart.

**15.** Device according to claim 1, **characterized in that** it comprises a cover (50) having openings (53, 55) for the circulation of the fluid.

**Patentansprüche**

**1.** Vorrichtung zur Detektion physikalischer Variablen einer Flüssigkeit mit einer Dielektrizitätskonstante, wobei die Vorrichtung (1) einen Körper (2) einschließt, der verbunden ist mit wenigstens:

- einem ersten Sensorelement der Art, die einen Kondensator umfasst, um wenigstens den Füllstand der Flüssigkeit im Innern eines jeweiligen Behälters zu ermitteln, und
- einem zweiten Sensorelement der Art, die einen Kondensator umfasst, um wenigstens einen Referenzparameter der Flüssigkeit zu ermitteln, wie z. B. ihre Dielektrizitätskonstante,

wobei

- jedes Sensorelement wenigstens zwei Elektroden (22, 23, 24; 22", 23", 40) aufweist, die ausgehend von wenigstens einem metallischen Plättchen erhalten werden, das zwei Hauptseiten und eine Umfangsfläche aufweist, die durch die Dicke des Plättchens definiert ist, wobei jede Elektrode (22, 23, 24; 22", 23", 40) wenigstens die Plattenoberfläche eines jeweiligen Kondensators definiert, die der Plattenoberfläche eines homologen Kondensators der anderen Elektrode (22, 23, 24; 22", 23", 40) gegenübersteht,
- die Elektroden (22, 23; 22", 23") von einem des ersten und zweiten Sensorelements wenigstens die Plattenoberfläche eines jeweiligen Kondensators aufweisen, die in der Umfangsfläche eines jeweiligen Plättchens gebildet ist, wobei die Elektroden des einen Sensorelements eine erste und eine zweite Elektrode (22, 23; 22", 23") der Vorrichtung (1) umfassen, die Plattenoberfläche des Kondensators jeder der ersten und zweiten Elektrode (22, 23; 22", 23") eine Vielzahl von Vorsprüngen (22C, 23C) besitzt, die aufeinanderfolgend entlang einer Längsausdehnung der Elektrode angeordnet sind, und die erste und zweite Elektrode (22, 23; 22", 23") so angeordnet sind, dass die Vorsprünge (22C) der ersten Elektrode (22; 22") zu den Vorsprüngen (23C) der zweiten Elektrode (23; 23") koplanar und ineinandergreifend sind,
- das erste und zweite Sensorelement wenigstens die erste Elektrode (22; 22") gemeinsam haben,

**dadurch gekennzeichnet, dass**

- bei den Elektroden (22, 24; 22", 40) des anderen des ersten und zweiten Sensorelements wenigstens die Plattenoberfläche eines jeweiligen Kondensators in einer Hauptseite eines jeweiligen Plättchens definiert ist, wobei die Elektroden (22, 24; 22", 40) des anderen Sensorelements eine dritte Elektrode (24; 40) der Vorrichtung (1) umfassen und die dritte Elektrode (24; 40) wenigstens einen Plattenbereich (24', 24"; 41 B) an einer Hauptseite davon aufweist, welche die Plattenoberfläche eines jeweiligen Kondensators des anderen Sensorelements bildet;
- die erste Elektrode (22; 22") ebenfalls einen Plattenbereich (22D) mit wenigstens einer Hauptseite umfasst, welche die Plattenoberfläche eines jeweiligen Kondensators des anderen Sensorelements bildet, und
- die erste und die dritte Elektrode (22, 24; 22", 40) so angeordnet sind, dass die jeweiligen Plattenbereiche (22D, 24', 24"; 22D, 41 B) im Wesentlichen zueinander parallel oder gleichmäßig beabstandet sind.

**2.** Vorrichtung nach Anspruch 1, **gekennzeichnet durch** das Umfassen einer Tragkonstruktion, die aus einem elektrisch isolierenden Material (21, 21") hergestellt und ausgelegt ist,

- um die erste und die zweite Elektrode (22, 23; 22", 23") im Wesentlichen auf einer gleichen Liegefläche zu halten, und
- um den wenigstens einen Plattenbereich (24', 24"; 41 B) der dritten Elektrode (24; 40) im Wesentlichen parallel oder gleichmäßig beabstandet zum Plattenbereich (22D) der ersten Elektrode (22; 22") zu halten.

**3.** Vorrichtung nach Anspruch 2, **dadurch gekennzeichnet, dass** die Tragkonstruktion aus einem thermoplastischen Material hergestellt ist, das um die erste, zweite und dritte Elektrode (22, 23, 24) gespritzt ist.

**4.** Vorrichtung nach Anspruch 1, **dadurch gekennzeichnet, dass** der Plattenbereich (22D) und die Vielzahl von Vorsprüngen (22C) der ersten Elektrode (22; 22") auf einer gleichen Ebene liegen.

**5.** Vorrichtung nach Anspruch 1, **dadurch gekennzeichnet, dass** die dritte Elektrode (24) zwei Plattenbereiche (24'; 24") aufweist, zwischen denen der Plattenbereich (22D) der ersten Elektrode (22; 22") angeordnet ist.

**6.** Vorrichtung nach Anspruch 5, **dadurch gekennzeichnet, dass** die dritte Elektrode (24) durch eine Anzahl getrennter Teile (24A-24B, 24', 24") gebildet ist, die elektrisch und mechanisch aneinander angeschlossen sind.

**7.** Vorrichtung nach Anspruch 6, **dadurch gekennzeichnet, dass** wenigstens einige der getrennten Teile (24A-24B, 24', 24") gegenseitig verschweißt sind.

**8.** Vorrichtung nach Anspruch 1, **dadurch gekennzeichnet, dass** wenigstens einer der Plattenbereiche (22D, 24', 24"; 22D, 41 B) wenigstens einen entsprechenden Durchgangsschlitz (22D'; 41 C) aufweist.

**9.** Vorrichtung nach Anspruch 1, **dadurch gekennzeichnet, dass** die Vorsprünge (22C, 23C) wie zugespitzte Zähne gestaltet sind.

**10.** Vorrichtung nach Anspruch 1, **dadurch gekennzeichnet, dass** das erste Sensorelement einen Rahmen (21") für die erste und zweite Elektrode (22", 23") umfasst, der Sitze oder Führungen (F, S) definiert, in denen die dritte Elektrode (40) wenigstens teilweise eingesetzt oder fixiert ist.

**11.** Vorrichtung nach Anspruch 1, **dadurch gekennzeichnet, dass** dem Körper (2) weiterhin Sensormittel zum Ermitteln der Temperatur (60) der Flüssigkeit zugeordnet sind.

**12.** Vorrichtung nach Anspruch 1, **dadurch gekennzeichnet, dass** dem Körper (2) weiterhin Sensormittel zum Ermitteln der Viskosität der Flüssigkeit zugeordnet sind, die einen Schwingungserzeuger (70) umfassen.

**13.** Vorrichtung nach Anspruch 2, **dadurch gekennzeichnet, dass** die Tragkonstruktion (21) wenigstens zwei parallele Längsständer (21B, 21C) und einen Querträger (21A, 21D, 21E) umfasst.

**14.** Vorrichtung nach Anspruch 13, **dadurch gekennzeichnet, dass** die Tragkonstruktion einen Basis-Querträger (21A) umfasst, von dem wenigstens zwei Verlängerungen zur Koppelung (25, 26) an den Körper (2) abgehen.

**15.** Vorrichtung nach Anspruch 1, **dadurch gekennzeichnet, dass** sie eine Abdeckung (50) mit Öffnungen (53, 55) für die Zirkulation der Flüssigkeit umfasst.

## Revendications

**1.** Dispositif de détection des variables physiques d'un fluide possédant une constante diélectrique, le dispositif (1) comprenant un corps (2) associé à au moins

- un premier élément de capteur, du type comprenant un condensateur, pour détecter au moins le niveau du fluide à l'intérieur d'un récipient respectif, et
- un second élément de capteur, du type comprenant un condensateur, pour détecter au moins un paramètre de référence du fluide, tel que sa constante diélectrique,

dans lequel

- chaque élément de capteur possède au moins deux électrodes (22, 23, 24 ; 22", 23", 40) obtenues à partir d'au moins une lame métallique possédant deux faces principales et une surface périphérique qui est définie par l'épaisseur de la lame, chaque électrode (22, 23, 24 ; 22", 23", 40) définissant au moins une surface de plaque de condensateur respectif faisant face à une surface de plaque de condensateur homologue de l'autre électrode (22, 23, 24 ; 22", 23", 40)
- les électrodes (22, 23 ; 22", 23") d'un desdits premier et second éléments de capteur possèdent au moins une surface de plaque de condensateur respectif qui est formée dans la surface périphérique d'une lame respective, les électrodes dudit un élément de capteur comprenant une première et une seconde électrodes (22, 23 ; 22", 23") du dispositif (1), la surface de plaque de condensateur de chacune desdites première et secondes électrodes (22, 23 ; 22", 23") possédant une pluralité de projections (22C, 23C) successivement disposées le long d'une extension longitudinale de l'électrode, lesdites première et seconde électrodes (22, 23 ; 22", 23") étant disposées de manière à ce que les projections (22C) de la première électrode (22 ; 22") soient co-planaires et interdigitées avec les projections (23C) de la seconde électrode (23 ; 23"),
- le premier et le second éléments de capteur possèdent au moins en commun ladite première électrode (22 ; 22"),

**caractérisé en ce que**

- les électrodes (22, 24 ; 22", 40) de l'autre desdits premier et second éléments de capteur possèdent au moins une surface de plaque de condensateur respectif définie dans une face principale d'une lame respective, les électrodes (22, 24 ; 22", 40) dudit autre élément de capteur comprenant une troisième électrode (24 ; 40) du dispositif (1), la troisième électrode (24 ; 40) comprenant au moins une portion de plaque (24', 24" ; 41B) dont une face principale forme une surface de plaque de condensateur respectif dudit autre élément de capteur ;

- ladite première électrode (22 ; 22") comprend également une portion de plaque (22D) possédant au moins une face principale qui forme une surface de plaque de condensateur respectif dudit autre élément de capteur, et

- la première et la troisième électrodes (22, 24 ; 22", 40) sont disposées de manière à ce que les portions de plaque respectives (22D, 24', 24" ; 22D, 41B) soient essentiellement parallèles ou uniformément espacées les unes des autres.

2. Dispositif selon la revendication 1, **caractérisé en ce qu'**il comprend une structure de support faite d'un matériau électriquement isolant (21, 21"), conçue

    - pour maintenir essentiellement sur un même plan la première et la seconde électrodes (22, 23 ; 22", 23"), et
    - pour maintenir ladite au moins une portion de plaque (24', 24" ; 41B) de la troisième électrode (24 ; 40) essentiellement parallèle ou uniformément espacée de la portion de plaque (22D) de la première électrode (22 ; 22").

3. Dispositif selon la revendication 2, **caractérisé en ce que** la structure de support est faite d'un matériau thermoplastique moulé sur la première, la seconde et la troisième électrodes (22, 23, 24).

4. Dispositif selon la revendication 1, **caractérisé en ce que** la portion de plaque (22D) et la pluralité de projections (22C) de la première électrode (22 ; 22") se trouvent sur un même plan.

5. Dispositif selon la revendication 1, **caractérisé en ce que** la troisième électrode (24) comprend deux portions de plaque (24', 24") entre lesquelles la portion de plaque (22D) de la première électrode (22 ; 22") est placée.

6. Dispositif selon la revendication 5, **caractérisé en ce que** la troisième électrode (24) est formée par un certain nombre de parties séparées (24A-24B, 24', 24") électriquement et mécaniquement connectées les unes aux autres.

7. Dispositif selon la revendication 6, **caractérisé en ce qu'**au moins certaines desdites parties séparées (24A-24B, 24', 24") sont réciproquement soudées.

8. Dispositif selon la revendication 1, **caractérisé en ce qu'**au moins une desdites portions de plaque (22D, 24', 24" ; 22D, 41B) possède au moins une fente de passage (22D' ; 41C).

9. Dispositif selon la revendication 1, **caractérisé en ce que** lesdites projections (22C, 23C) ont une forme de dents pointues.

10. Dispositif selon la revendication 1, **caractérisé en ce que** ledit un élément de capteur comprend un cadre (21") pour la première et la seconde électrodes (22", 23") définissant des assises ou des guides (F, S) dans lesquels la troisième électrode (40) est au moins partiellement insérée ou fixée.

11. Dispositif selon la revendication 1, **caractérisé en ce que** les moyens de détection pour détecter la température (60) du fluide sont en outre associés audit corps (2).

12. Dispositif selon la revendication 1, **caractérisé en ce que** les moyens de détection pour détecter la viscosité du fluide sont en outre associés audit corps (2), comprenant un générateur d'oscillation (70).

13. Dispositif selon la revendication 2, **caractérisé en ce que** la structure de support (21;) comprend au moins deux montants longitudinaux parallèles (21B, 21C) et une pièce transversale (21A, 21D, 21E).

14. Dispositif selon la revendication 13, **caractérisé en ce que** la structure de support comprend une pièce transversale de base (21A) à partir de laquelle partent au moins deux extensions pour le couplage (25, 26) audit corps (2).

15. Dispositif selon la revendication 1, **caractérisé en ce qu'**il comprend un couvercle (50) possédant des ouvertures (53, 55) pour la circulation du fluide.

Fig. 1

**20**

**21**

**1**

**11A**

**11B**

**D**

**2**

**TC**

**5**

Fig. 2

**20**

**22**

**23**

**1**

**24**

**27**

**27**

**11A**

**2**

**5**

**55**

**51**

**50**

**54**

**53**

**52**

**20**

**23**

**22**

Fig. 3

**1**

**70**

**24**

**4**

**4**

**2**

**5**

**11A**

**Fig. 4**

**Fig. 5**     **Fig. 6**     **Fig. 7**

**Fig. 8**

**Fig. 9**

**Fig. 10**

**Fig. 11**

**Fig. 12**

EP 1 462 775 B1

**Fig. 13**

23C

23B

23

23A

**Fig. 14**

22C

22B

22D

22F

22

22A

23

22

B

24B

24A

**Fig. 16**

**Fig. 15**

24B

24A

24I 24G 24'

24H

24H

24I

24"

24G

**Fig. 17**

80

81

90

91

**Fig. 18**

81D

81F

81

81B

81C

81A

81C

81E

80

**Fig. 19**

91F

91E

90

91B

91C

91D'

91D

91D''

91C

91D'

91A

91D

91

91D''

**Fig. 20**

**Fig. 21**

**Fig. 22**

81

80

90

20

**Fig. 23**

20

21B

23C

28

21D

22C

24'

23A

24A

26

21A

25

27

21C

22A

**Fig. 24**     **Fig. 25**     **Fig. 27**

22C

22B

22D

22D'

22''

22A

23C

23B

23''

23A

22''

23''

22D

41C

41B

40

41A

**Fig. 26**

**Fig. 29**

**Fig. 30**

**Fig. 28**

**Fig. 31**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 02084228 A

- US 5051921 A